# EUROPEAN PATENT APPLICATION

(11) **EP 4 238 641 A1**
(43) Date of publication of application: **06.09.2023**
(21) Application number: 21885257.2
(22) Date of filing: 28.10.2021
(51) Int. Cl.: B01J 14/00, C07C 29/04, C11C 3/10

(54) **LIQUID-LIQUID MIXER, LIQUID-LIQUID REACTION APPARATUS COMPRISING LIQUID-LIQUID MIXER, AND LIQUID-LIQUID REACTION METHOD USING LIQUID-LIQUID MIXER**

(30) Priority: 28.10.2020 CN 202011172318
(71) Applicant: China Petroleum & Chemical Corporation, Beijing 100728 (CN); Sinopec Dalian Research Institute of Petroleum and Petrochemicals Co., Ltd., Lushunkou District Dalian, Liaoning 116045 (CN)
(72) Inventor: ZHOU, Feng, Dalian Liaoning 116045 (CN); MA, Huixia, Dalian Liaoning 116045 (CN); YANG, Xiuna, Dalian Liaoning 116045 (CN); QIAO, Kai, Dalian Liaoning 116045 (CN); ZHANG, Shumei, Dalian Liaoning 116045 (CN); JIANG, Rui, Dalian Liaoning 116045 (CN); LI, Lanpeng, Dalian Liaoning 116045 (CN)
(74) Representative: Hoffmann Eitle
(86) International application number: PCT/CN2021/127019
(87) International publication number: WO 2022/089530

(57) **Abstract**

The present disclosure provides a microchannel liquid-liquid mixing device, comprising a microchannel component and a shell, wherein the microchannel component is fixed inside the shell, wherein an inlet is provided at one end of the shell for feeding at least two reaction liquid phases, and an outlet is provided at the other end for discharging a mixed material; said microchannel component comprises multiple stacked sheets and oleophilic fiber filaments and hydrophilic fiber filaments filled in the crevices between adjacent sheets, wherein the fiber filaments form several microchannels between them, and the fiber filaments are clamped and fixed by the sheets. The microchannel liquid-liquid mixing device is used for at least two reaction liquid phases to form a mixed material, and the at least two reaction liquid phases are cut by fiber filaments and mixed in the microchannel mixing device to form a mixed material. The present invention also discloses the liquid-liquid reaction apparatus and liquid-liquid reaction process comprising the above microchannel liquid-liquid mixing device, such as an olefin hydration reaction apparatus and an olefin hydration process, and a reaction apparatus and process for producing biodiesel with transesterification.

## Description

### Technical Field

The present invention belongs to the field of organic chemical industries. Specifically, the present invention relates to a liquid-liquid mixer, a liquid-liquid reaction apparatus including the same, and a liquid-liquid reaction process using the same, in particular to an olefin hydration reaction apparatus and an olefin hydration process, a biodiesel production apparatus, and a process for producing biodiesel through transesterification.

### Background Technology

The olefin hydration process is a three-phase catalytic reaction of liquid (olefin)-liquid (water)-solid (catalyst), so the reaction rate and the conversion rate are greatly affected by liquid-liquid mass transfer. However, the low mutual solubility between the two liquid phases (i.e. the olefin phase and the aqueous phase) limits the reaction efficiency and the production capacity of the olefin hydration process. In olefin hydration reactions, typical and important olefin hydration reactions include cyclohexene hydration to cyclohexanol, n-butene hydration to sec-butanol, isobutene hydration to tert-butanol, and isopentene hydration to tert-pentanol.

In the process of cyclohexene hydration to cyclohexanol, the reactor form of the direct cyclohexene hydration production apparatus currently used in industry is a two-stage in-series fully mixed reactor, with a low once-through conversion (generally<10%), resulting in a large number of unreacted cyclohexene and cyclohexanol being separated by multiple cycles of fine distillation, resulting in high energy consumption.

CN109651081A proposes a reactive distillation method and apparatus for preparing cyclohexanol through the hydration of cyclohexene. The method involves adding a phase transfer catalyst to the reaction liquid to form a slurry solution of cyclohexene, water, the catalyst, and the phase transfer catalyst. In the reactive distillation column, cyclohexene hydration reaction occurs to generate cyclohexanol. Although the reactive distillation method can theoretically greatly improve the conversion rate of cyclohexene and reduce the system energy consumption, however the hydration reaction rate is low.

US3257469 uses a polar organic solvent to increase the mutual solubility of olefin and water, and improves the conversion rate of C5 olefins by increasing the diffusion rate of reactant molecules towards the catalyst surface and the diffusion rate of products into the solvent.

US4182920 uses a three-stage olefin hydration reactor, with a reaction temperature of 30-80°C, a reaction pressure of 0.46-1.4MPa (absolute pressure), a weight ratio of water to pentene of 0.59-1.18, and a weight ratio of acetone to pentene of 4.18-7.85. The reaction rate is still very slow.

In the process of preparing tert-butanol by hydration of isobutene, the mutual solubility of hydrocarbons such as isobutene with water is small, and when mixed in liquid state, it is easy to form two liquid phases. The key technology for isobutene hydration is to form a homogeneous dispersion or solution of C4 mixed hydrocarbons and water, in order to improve the conversion rate of the reaction.

CN1304917A adopts a method of feeding isobutylene and water in a countercurrent manner into the column, and the product tert-butanol is continuously discharged from the column to increase the concentration difference between the two phases, improve the reaction rate and the isobutylene conversion rate. However, this method has problems such as high water ratio, low space velocity, and difficulty in catalyst molding.

CN02151547.6 (CN1511815A) provides a method for preparing tert-butanol from a mixed C4 raw material containing isobutene by the hydration reaction. In the presence of a non-ionic surfactant and a catalyst, isobutene in the mixed C4 fraction reacts with water in a catalytic distillation column to produce tert-butanol. The preparation method of tert-butanol provided by the invention greatly improves the conversion rate of isobutene and the selectivity of tert-butanol, and reduces the cost of producing tert-butanol.

CN98812676.1 (CN1283174A) provides a method for producing tert-butanol. In order to increase the mutual solubility of mixed C4 and water, tert-butanol is added to the reaction raw material, and the conversion rate of the reaction can reach 80-90%. This method relies on the centrifugal pump to forcibly mix three phases (tert-butanol, water, and liquid phase C4 hydrocarbons) to promote the dissolution. However, in reality, the method of using the centrifugal pump for the forced mixing only performs a macroscopic mixing of phases, and it is difficult to achieve the uniform mixing at the microcosmic level.

Biodiesel has the advantages such as good engine startability under low temperatures, low sulfur content, no aromatic hydrocarbons that cause environmental pollution, high flash point, good safety performance, high cetane number and good lubrication performance. At present, the transesterification method is mostly used in the production of biodiesel in industry, which uses low-carbon alcohols such as methanol to replace the glycerol in the triglyceride in the raw oil by transesterification under the action of catalyst to obtain biodiesel. Transesterification is the core of the whole process. The existing technology is mainly based on the insoluble system of triglyceride and methanol. It is difficult to mix and dissolve the two phases, which causes many problems in the transesterification process, such as low liquid-liquid mass transfer reaction rate, easy phase separation, and long time for the reaction to reach the established conversion rate.

In order to solve the above problems, CN1919973A utilizes a loop turbulence reactor to form a continuous device for preparing biodiesel. The raw materials enter from the bottom of the loop turbulence reactor and react in the loop turbulence reactor. The reacted mixture is discharged from the top of the reactor, and a portion of the materials return to the recycle mixing pump and enter the reactor again. The purpose is to achieve multiple forced mixing cycles of the raw materials, which requires a significant energy consumption and a special reactor structure to be achieved.

CN101550349 adopts the supercritical technology to prepare biodiesel. This process turns the appropriate molar ratio of oil and methanol into a homogeneous phase under supercritical conditions, and conducts the transesterification. The controlled pressure is 8-40 MPa, and the temperature is 300-450°C. This invention shortens the time for the conversion of biological oil/fat into biodiesel, and improves the reaction efficiency. However, the biological process of supercritical biodiesel needs to be carried out under high temperature and high pressure. Both energy consumption and equipment investment are high. High temperature conditions can easily lead to problems such as coking and blockage.

CN1952046A proposes a process for producing biodiesel by transesterification, which comprises passing the oil raw material and low-carbon alcohol that participate in the reaction into the transesterification reactor equipped with an ultrasonic emission device according to the reaction metering ratio to react under appropriate conditions. The purpose of this inventive process is to improve the mutual solubility of the oil raw material and the alcohol through the introduction of ultrasound, increase the mass transfer reaction rate of immiscible two phases, and shorten the reaction time. However, the ultrasonic generation device in this process is difficult to industrialize, and it is difficult for the entire mixing system of materials to achieve the same degree of uniformity on industrial apparatuses.

CN201625532U discloses a high shear emulsification reaction apparatus for preparing biodiesel from waste oil and fat, which includes a reaction kettle, an electrical motor, and an emulsifying machine. The electric motor is fixed outside the reaction kettle. The emulsifying machine includes an outer casing, a rotor, and a stator. The outer casing, the rotor, and the stator are all coaxial hollow cylinders. One end of the outer casing passes through the outer house of the reaction kettle and is fixed to the outer house of the electrical motor, and the other end is connected to the stator. One end of the main shaft of the electric motor is fixedly connected to the rotor. Multiple permeation grooves running through the outer walls of the rotor and stator are arranged along the axial direction on the outer walls. The rotor is located in the hollow inner cavity of the stator. The utility model mainly aims to perform the strong shear emulsification on the reaction materials in order to improve material mixing. On the one hand, the degree of improvement through mechanical shear is limited, and on the other hand, the high shear emulsification reactor is a fully mixed reactor, which has low once-through conversion and low production efficiency.

For the olefin hydration reaction (generally cyclohexene hydration to cyclohexanol, n-butene hydration to sec-butanol, isobutene hydration to tert-butanol, and isopentene hydration to tert-pentanol), the mutual solubility of olefin and water greatly affects the mass transfer reaction rate. In the biodiesel reaction procedure, the immiscibility between triglyceride and low-carbon alcohol has caused many problems such as low transesterification mass transfer reaction rate, easy phase separation, long reaction residence time, and low raw material conversion rate. Although at current, researchers have proposed many new processes and devices, the transesterification reaction has not been significantly improved.

For liquid-liquid reactions, poor mutual solubility between reaction materials can result in that the reaction has low once-through conversion and slow reaction rate. Therefore, with respect to this type of liquid-liquid reaction, it is necessary to provide a new and effective process and apparatus to further solve the above problems.

### Summary of the Invention

Aiming at the shortcomings of the existing technology, the present invention proposes a liquid-liquid mixer. The liquid-liquid mixer of the present invention can be widely applied to liquid-liquid reactions with poor mutual solubility between reaction materials. The liquid-liquid reaction of the present invention refers to a reaction that includes at least two liquid phases, including but not limited to liquid-liquid reactions and liquid-liquid-solid reactions. In particular, the liquid-liquid reaction of the present invention does not include a gas phase feed stream.

In one aspect, the present invention provides a microchannel liquid-liquid mixing device, comprising a microchannel component and a shell, wherein the microchannel component is fixed inside the shell, wherein an inlet is provided at one end of the shell for feeding at least two reaction liquid phases, and an outlet is provided at the other end for discharging a mixed material; said microchannel component comprises multiple stacked sheets and oleophilic and hydrophilic fiber filaments filled in the crevices between adjacent sheets, wherein the fiber filaments form several microchannels between them, and the fiber filaments are clamped and fixed by the sheets. The microchannel liquid-liquid mixing device is used for at least two reaction liquid phases to form a mixed material, and the at least two reaction liquid phases are cut by fiber filaments and mixed in the microchannel mixing device to form a mixed material.

In another aspect, the present invention provides a liquid-liquid reaction apparatus, which includes a microchannel mixing device I, a microchannel mixing device II, and a reactor;
said microchannel mixing device I has a tube-shell type structure, and a bundle of inorganic membrane tubes is arranged inside the shell; the inlet end of the bundle of inorganic membrane tubes is communicated with the first liquid phase feeding pipeline, the cavity in the shell outside of the bundle of inorganic membrane tubes is communicated with a second liquid phase feeding pipeline, and the outlet end of the bundle of inorganic membrane tubes is an outlet for a mixed material I; the microchannel mixing device I is used for feeding a first liquid phase and a second liquid phase to form a mixed material I, the second liquid phase diffuses into the first liquid phase inside the inorganic membrane tube through porous channels in the tube wall of the inorganic membrane tube from the cavity in the shell, and under the action of the shearing force of the first liquid phase having a high flow rate in the tube, two liquid phases forms a homogeneous mixed material I, which is used as the main reaction feed; preferably, a control device is provided in the microchannel mixing device I so that the ratio by mole or by weight of the first liquid phase to the second liquid phase is greater than or less than (preferably greater than) the theoretical ratio by mole or by weight of the first liquid phase to the second liquid phase of the reaction;
Said microchannel mixing device II is a microchannel liquid-liquid mixing device of the present invention, which includes a microchannel component and a shell, an inlet is provided at one end of the shell for feeding the first liquid phase and the second liquid phase, and an outlet is provided at the other end for discharging a mixed material II; said microchannel component comprises multiple stacked sheets and oleophilic and hydrophilic fiber filaments filled in the crevices between adjacent sheets, wherein the fiber filaments form several microchannels between them, and the fiber filaments are clamped and fixed by the sheets; the microchannel mixing device II is used for the first liquid phase and the second liquid phase to form the mixed material II, and the first liquid phase and the second liquid phase are cut by fiber filaments and mixed in the microchannel mixing device II to form the mixed material II; wherein preferably a control device is provided in the microchannel mixing device II so that the ratio by mole or by weight of the first liquid phase to the second liquid phase is not greater than or not less than (preferably not greater than) the theoretical ratio by mole or by weight of the first liquid phase to the second liquid phase of the reaction.

The top, the bottom, or the side of the reactor is provided with feed inlet(s), while the bottom, the top, or the side is provided with discharge outlet(s). The reactor body is provided with an inlet for the mass transfer-enhancing material. In principle, the inlet for the mass transfer-enhancing material can be provided at any position within the reactor. The outlet for the mixed material I of the microchannel mixing device I is connected to the feed inlet through pipeline(s), and the outlet for the mixed material II of the microchannel mixing device II is connected to the inlet for the mass transfer-enhancing material.

In still another aspect, the present invention provides a process for performing the liquid-liquid reaction by using the liquid-liquid reaction apparatus of the present invention. The liquid-liquid reaction includes: emulsion polymerization and suspension polymerization in polymer chemical industry, such as the olefin polymerization in organic solvent; the olefin hydration reaction; the transesterification to produce biodiesel; the oil/fat hydrolysis; and other reactions such as nitrification, sulfonation, and alkylation.

Specifically, the present invention provides an olefin hydration reaction apparatus, comprising a microchannel mixing device I, a microchannel mixing device II, and an olefin hydration reactor;
said microchannel mixing device I has a tube-shell type structure, and a bundle of inorganic membrane tubes is arranged inside the shell; the inlet end of the bundle of inorganic membrane tubes is communicated with an aqueous phase pipeline, the cavity in the shell outside of the bundle of inorganic membrane tubes is communicated with an olefin phase pipeline, and the outlet end of the bundle of inorganic membrane tubes is an outlet for a mixed material I; the microchannel mixing device I is used for feeding the olefin phase and feeding the aqueous phase to form a mixed material I, the olefin phase diffuses into the aqueous phase inside the inorganic membrane tube through porous channels in the tube wall of the inorganic membrane tube from the cavity in the shell, and under the action of the shearing force of the aqueous phase having a high flow rate in the tube, two phases form a homogeneous mixed material I, which is used as the main reaction feed; wherein the aqueous phase/olefin phase ratio ≥1;
said microchannel mixing device II includes a microchannel component and a shell, the microchannel component is fixed inside the shell, an inlet is provided at one end of the shell for feeding the olefin phase and the aqueous phase, and an outlet is provided at the other end for discharging a mixed material II; said microchannel component comprises multiple stacked sheets and oleophilic and hydrophilic fiber filaments filled in the crevices between adjacent sheets, wherein the fiber filaments form several microchannels between them, and the fiber filaments are clamped and fixed by the sheets; the microchannel mixing device II is used for the olefin phase and the aqueous phase to form a mixed material II, and the olefin phase and the aqueous phase are cut by fiber filaments and mixed in the microchannel mixing device II to form a mixed material II; wherein the aqueous phase/olefin phase ratio is <1;
The bottom of the olefin hydration reactor is provided with a feed inlet, the top is provided with a discharge outlet, and the side is provided with an inlet for the mass transfer-enhancing material. Several catalyst beds are provided in the reactor, and the inlet for the mass transfer-enhancing material is provided at any position within the reactor, preferably between adjacent catalyst beds in a more convenient manner for implementation; the catalyst bed(s) are filled with an olefin hydration catalyst. The outlet for the mixed material I of the microchannel mixing device I is connected to the feed inlet through pipeline(s), and the outlet for the mixed material II of the microchannel mixing device II is connected to the inlet for the mass transfer-enhancing material.

With the olefin hydration reaction apparatus and the olefin hydration process of the present invention, by improving the mixing state of olefin and water and the mixed feeding manner, the prevent invention strengthens the mass transfer of the entire reaction process, improves the olefin hydration reaction rate and the raw material once-through conversion, reduces the overall aqueous phase/olefin phase ratio, and improves the production efficiency of the olefin hydration apparatus. The aqueous phase/olefin phase ratio mentioned in the present invention refers to the ratio by weight of the aqueous phase to the olefin phase. The overall aqueous phase/olefin phase ratio refers to the mass ratio of the total addition amount of the aqueous phase to the total addition amount of olefin in the olefin hydration reaction apparatus.

Specifically, the present invention also provides a reaction apparatus for producing biodiesel by transesterification, which comprises a microchannel mixing device I, a microchannel mixing device II and a transesterification reactor;
said microchannel mixing device I has a tube-shell type structure, a bundle of inorganic membrane tubes is arranged inside the shell; the inlet end of the bundle of inorganic membrane tubes is communicated with pipeline(s) for low carbon alcohol and liquid catalyst, the cavity in the shell outside of the bundle of inorganic membrane tubes is communicated with the triglyceride pipeline, the outlet end of the bundle of inorganic membrane tubes is the outlet for the mixed material I; the microchannel mixing device I is used for feeding triglyceride and feeding the low carbon alcohol and liquid catalyst to form a mixed material I, the triglyceride diffuses into the low carbon alcohol and liquid catalyst inside the inorganic membrane tube through porous channels in the tube wall of the inorganic membrane tube from the cavity in the shell, and under the action of the shearing force of the low carbon alcohol and liquid catalyst having a high flow rate in the tube, two phases form a homogeneous mixed material I, which is used as the main reaction feed; wherein the molar ratio of low carbon alcohol to triglyceride ≥3;
Said microchannel mixing device II includes a microchannel component and a shell, the microchannel component is fixed inside the shell, an inlet is provided at one end of the shell for feeding the low carbon alcohol and liquid catalyst and triglyceride, and an outlet is provided at the other end for discharging the mixed material II; said microchannel component comprises multiple stacked sheets and oleophilic and hydrophilic fiber filaments filled in the crevices between adjacent sheets, wherein the fiber filaments form several microchannels between them, and the fiber filaments are clamped and fixed by the sheets; the microchannel mixing device II is used for the low carbon alcohol and liquid catalyst and the triglyceride to form a mixed material II, and the low carbon alcohol and liquid catalyst and the triglyceride are cut by fiber filaments and mixed in the microchannel mixing device II to form a mixed material II; wherein the molar ratio of low carbon alcohol to triglyceride is <3.

The top, the bottom, or the side of the transesterification reactor is provided with feed inlet(s), while the bottom, the top, or the side is provided with discharge outlet(s). The reactor body is provided with inlet(s) for the mass transfer-enhancing material. In principle, the inlet for the mass transfer-enhancing material can be provided at any position within the reactor. The outlet for the mixed material I of the microchannel mixing device I is connected to the feed inlet through pipeline(s), and the outlet for the mixed material II of the microchannel mixing device II is connected to the inlet for the mass transfer-enhancing material.

With the reaction apparatus and process for producing biodiesel with transesterification of the present invention, by improving the mixing state of triglyceride and low carbon alcohol and the mixed feeding manner, the prevent invention strengthens the mass transfer rate of the entire reaction process, improves the transesterification reaction rate and the raw material once-through conversion, reduces the reaction time, and improves the production efficiency of the biodiesel production apparatus.

In the liquid-liquid reaction apparatus of the present invention (such as the olefin hydration reaction apparatus, the reaction apparatus for producing biodiesel with transesterification), the number of the microchannel mixing device I and the microchannel mixing device II can be set according to actual needs, generally 1-3 to meet the reaction needs. According to the apparatus of the present invention, the bundle of inorganic membrane tubes of said microchannel mixing device I can be of one or more of ceramic membrane, metal membrane, metal/ceramic composite membrane, alloy membrane, molecular sieve composite membrane, zeolite membrane glass membrane or the like; the tube wall of the inorganic membrane tube has a hole diameter of 10nm-1 µm; the second liquid phase (such as olefin phase, triglyceride phase ) in the mixed material I has a particle size d1 of 100-900 µm, preferably 300-500 µm.

In the microchannel mixing device II of the apparatus of the present invention, the microchannel component in the shell are divided into a feeding end and a discharging end along the direction of the crevice, wherein a feeding distribution space is provided between the material inlet and the feeding end, and a discharging distribution space is provided between the material outlet and the discharging end, except for the feeding end and the discharging end, all other ends of the microchannel component are connected to the shell in a sealed manner. said microchannel component comprises multiple stacked sheets and oleophilic fiber filaments and hydrophilic fiber filaments filled in the crevices between adjacent sheets, wherein the fiber filaments form several microchannels between them, and the fiber filaments are clamped and fixed by the sheets; the ratio by weight of the oleophilic fiber filament to the hydrophilic fiber filament filled in the crevices between said adjacent sheets is 1:50-1:1; said fiber filaments can be arranged in single or multiple layers, preferably 1-50 layers, and more preferably 1-5 layers, preferably the hydrophilic fiber filaments in any layer are uniformly distributed in the oleophilic fiber filaments; preferably the ratio by weight of the oleophilic fiber filament to the hydrophilic fiber filament in any layer is 1 :50-1: 1. When arranged in multiple layers, preferably the projection of two adjacent layers of fiber filaments along the vertical direction of the sheets forms a mesh structure; the mesh shape in a mesh structure can be an arbitrary shape, such as one or more of polygon, circle, ellipse, and the like; in each layer of fiber filaments, the distance between adj acent fiber filaments is generally 0.5 µm-50 µm, preferably arranged at equal intervals; the fiber filaments are arranged along any of the transverse, longitudinal or oblique direction of the surface of the sheet; the fiber filaments can have an arbitrary curve shape, preferably a periodically changing curve shape, such as a wavy shape and a serrated shape, preferably the fiber filaments in the same layer have the same shape, and more preferably, the fiber filaments in all layers have the same shape.

In the microchannel component, the fiber filaments generally have a diameter of 0.5-50 µm, preferably 0.5-5 µm, more preferably 0.5-1 µm.Said oleophilic fiber filament is generally at least one of a polyester fiber filament, a nylon fiber filament, a polyurethane fiber filament, a polypropylene fiber filament, a polyacrylonitrile fiber filament, a polyvinyl chloride fiber filament, or an oleophilically surface-treated (physically or chemically) fiber filament; Said hydrophilic fiber filament is generally selected from a high molecular polymer containing at least one hydrophilic group such as carboxyl (-COOH), amido (-CONH-), amino (-NH₂-), or hydroxyl (-OH) in its main chain or side chain, and the more hydrophilic groups they contain, the better their hydrophilicity. Commonly used fibers include polypropylene fiber, polyamide fiber, acrylic fiber, or a hydrophilically-treated (physically or chemically) fiber filament.

According to the microchannel component, the thickness of the sheet is generally 0.05mm-5mm, preferably 0.1-1.5mm. The material of the sheet is generally determined by the properties of the flowing material and the operating conditions, and can be any one of materials such as metal, ceramic, organic glass, or polyester. Stainless steel (such as SS30403, SS30408, SS32168, and SS31603) in the metal material is preferred. The shape of the sheet can be any one of rectangle, square, polygon, circle, ellipse, or sector, preferably rectangle or square. The size and amount of the sheets can be designed and adjusted according to the actual needs of the reaction.

In the apparatus of the present invention, the reactor can be of any type of reactor, such as a fixed bed reactor, a tank reactor, a column reactor, a tubular reactor, or an improved form of the aforementioned reactors. One or more reactors can be set as needed, and the reactors can be connected in parallel or series; at least one mixed material formed by the microchannel mixing device II is introduced as the mass transfer-enhancing material.

For the olefin hydration reaction, in the apparatus of the present invention, the reactor is a fixed bed reactor, and one or more reactors can be set as needed, with parallel or series connection between the reactors; each reactor should be equipped with at least one, preferably 1-4 catalyst beds; at least one mixed material formed by the microchannel mixing equipment II is introduced as the mass transfer-enhancing material.

For the transesterification reaction, in the apparatus of the present invention, the reactor can be of any type of reactor, such as a tank reactor, a column reactor, a tubular reactor, or an improved form of the aforementioned reactors. One or more reactors can be set as needed, and the reactors can be connected in parallel or series; at least one mixed material formed by the microchannel mixing device II is introduced as the mass transfer-enhancing material.

The present invention also provides an olefin hydration process, which includes: a mixed material I is formed by mixing an olefin phase and an aqueous phase with an aqueous phase/olefin phase ratio of ≥1 with the microchannel mixing device I and sent to the bottom of the olefin hydration reactor as the main reaction material; and a mixed material II is formed by mixing an olefin phase and an aqueous phase with an aqueous phase/olefin phase ratio of <1 with a microchannel mixing device II and introduced to the reactor as the mass transfer-enhancing material; the mixed material I and the mixed material II undergo the olefin hydration reaction in the catalyst bed(s), and the reaction products flow out from the outlet at the top of the reactor and enter the next separation unit.

For the olefin hydration process, according to the process of the present invention, the operation conditions of the microchannel mixing device I generally include: the temperature is normal temperature to 250°C, the pressure is 1.0-10.0 MPaG; the operation conditions of the microchannel mixing device II generally include: the temperature is normal temperature to 200°C, the pressure is 1.0-10.0 MPaG.

For the olefin hydration process, according to the process of the present invention, the olefin phase is generally any one of ethylene, propylene, n-butene, isobutene, isopentene, cyclohexene or the like.

For the olefin hydration process, according to the process of the present invention, the olefin hydration reactor generally adopts a form of bottom-in and top-out, which is conducive to the uniform contact of the olefin phase and the aqueous phase for the mass transfer.

For the olefin hydration process, according to the process of the present invention, the overall aqueous phase/olefin phase ratio of the olefin hydration reaction is determined by the type of olefin that undergoes the olefin hydration reaction and the difficulty of the reaction; in the microchannel mixing device I, the aqueous phase/olefin phase ratio by mass is generally 2:1-20:1. An appropriately higher aqueous phase/olefin phase ratio can guarantee the mass transfer reaction rate during the hydration process, but the reactor volume becomes larger. Here, as long as the water phase in the reactor is maintained to be greater than the olefin phase, it is sufficient. In the microchannel mixing device II, the aqueous phase/olefin phase ratio by mass is generally 1:20-1:1. The aqueous phase/olefin phase ratio in this material is relatively low, that is, the proportion/content of olefin is relatively high. As the mass transfer-enhancing material, it can quickly break through the phase interface and transfer to the catalyst surface to supplement the olefin consumed in the reaction, keeping a large amount of the homogeneous olefin/water phase (that is rich in olefin molecules) on the catalyst surface all along, thereby increasing the once-through conversion of the olefin hydration reaction.

For the olefin hydration process, according to the process of the present invention, in the mixed material I formed by said microchannel mixing device I, the olefin droplets have a particle size d1 of 100-900 µm and preferably have the disperse uniformity of ≥80%. At this time, when entering the catalyst bed of the reactor for the reaction, no phase separation between the olefin phase and the aqueous phase can be maintained during the reaction residence time, improving the mass transfer reaction rate, and achieving good reaction effect and good once-through conversion of olefin raw material; In the mixed material II formed by microchannel mixing device II, the olefin droplets have a particle size d2 of less than 100 µm, preferably 0.1-50 µm. When introduced as a mass transfer-enhancing material between catalyst beds, due to the small particle size of olefin and the concentration of olefin molecules, olefin molecules can quickly break through the phase interface and transfer to the catalyst surface when passing through the catalyst particles, playing a role of further enhancing the reaction mass transfer.

For the olefin hydration process, according to the process of the present invention, the addition amount of the mixed material II is 1wt%-30wt% of the total materials in the reactor (the total amount of the olefin phase and the aqueous phase); when the mixed material II is divided into multiple streams for the addition, it is preferable to gradually increase the addition amount of each stream along the flow direction of the materials in the reactor (for example, the addition amount of the latter stream increases by 5-20wt% relative to the addition amount of the former stream). Of course, the aqueous phase/olefin phase ratio can also be reduced or unchanged along the flow direction of the materials in the reactor. Here, as the olefin hydration reaction proceeds, the olefin molecules, especially those on the surface of the catalyst, are gradually consumed, and the mass transfer driving force of the reaction gradually decreases. However, since the amount of water in the reactor is much greater than that of the olefin phase, the low concentration of olefin molecules leads to a gradual reduction of olefin molecules on the surface of the catalyst. Therefore, by supplementing the mass transfer-enhancing material having a high olefin content between the catalyst beds, it can quickly break through the phase interface and transfer to the catalyst surface to timely supplement the olefin consumed in the reaction, thereby maintaining a relatively high reaction rate.

For the olefin hydration process, according to the process of the present invention, catalysts with acid catalytic functions, such as mineral acids, benzenesulfonic acids, ion exchange resins, molecular sieves, and other types of catalysts, can generally be used in the catalyst bed(s) of the olefin hydration reactor.

For the olefin hydration process, according to the process of the present invention, the conditions of the olefin hydration reaction generally comprise: the temperature is 80-250°C, the pressure is 1.0-10.0MPaG, and the space velocity is 0.1-3.0 h⁻¹. The required reaction conditions may vary depending on the olefin raw materials.

When conventional olefin/water mixing processes, mixing devices, or components with mixing functions in the existing technologies are used for mixing, there are problems of non-uniform mixing, unstable state, and ease of phase separation, resulting in the low mass transfer rate of the olefin hydration. Therefore, even at relatively high aqueous phase/olefin phase ratios, the once-through conversion is still very low, especially for the n-butene hydration, which has a once-through conversion of only 6%-8% when a conventional reactor is used. According to the olefin hydration apparatus and the reaction process of the present invention, most of the olefin and water are micro-mixed in a certain proportion with the microchannel mixing device I, so that the reaction feed can maintain two phases to be homogeneous without the phase separation during the reaction process in the reactor. In this case, the aqueous phase in the reactor is kept greater than the olefin phase, thereby achieving the continuous mass transfer between the olefin phase and the aqueous phase in the reactor; then a part of olefins and water are micro-mixed in another proportion with the microchannel mixing device II, so that the droplet size (d1) of the olefin phase in the formed mixed material I is less than the droplet size (d2) of the olefin phase in the mixed material II, and the droplet concentration degree of olefin phase in the mixed material I is less than the droplet concentration degree of the olefin phase in the mixed material II, Therefore, when introducing the mixed material II containing a higher concentration degree of the olefin phase into the catalyst bed, it can quickly break through the phase interface and promptly supplement the olefin phase consumed in the reaction, playing a role of enhancing the mass transfer, improving the reaction rate of the olefin hydration and the once-through conversion of raw materials, which is beneficial for reducing the aqueous phase/olefin phase ratio, reducing the amount of the reactors or the reaction residence time, and improving the production efficiency of the olefin hydration apparatus. Here, the droplet concentration degree of the olefin phase refers to the amount of olefin micro-droplets dispersed in the aqueous phase per unit volume. At the same time, the special structure of the microchannel components in the microchannel mixing device II, i.e. a certain proportion of metal fiber filaments filled in the crevices between said adjacent sheets, can adhere and spread the water in the mixed feed of olefins and water along the surface of the fiber filaments. The repeated forced cutting by fiber filaments produces micron-sized particles. The fully mixed material contains the concentrated olefin droplets. Olefin droplets having a smaller size used as the mass transfer-enhancing material can quickly break through the phase interface and promptly supplement the olefin phase consumed in the reaction, play a role of enhancing the mass transfer, and reducing the aqueous phase/olefin phase ratio.

The present invention also provides a transesterification process, which includes: a mixed material I is formed by mixing two phases of low carbon alcohol and triglyceride having the molar ratio of low carbon alcohol to triglyceride of ≥3 with the microchannel mixing device I and sent to the transesterification reactor as the main reaction material; and a mixed material II is formed by mixing two phases of low carbon alcohol and triglyceride having the molar ratio of low carbon alcohol to triglyceride of <3 with a microchannel mixing device II and introduced to the reactor as the mass transfer-enhancing material; the mixed material I and the mixed material II undergo the transesterification reaction in the reactor, and the reaction products flow out from the outlet of the reactor and enter the separation unit. For the transesterification process, according to the process of the present invention, the operation conditions of the microchannel mixing device I generally include: the temperature is normal temperature to 150°C, the pressure is 0.5-3.0 MPaG; the operation conditions of the microchannel mixing device II generally include: the temperature is normal temperature to 150°C, the pressure is 0.5-3.0 MPaG. In particular, the amount of the liquid catalyst is 0.5%-10% of the amount of the oil/fat raw material.

For the transesterification process, according to the process of the present invention, the oil/fat raw material is a triglyceride, mainly derived from animal oils or vegetable oils, including the oil and fat having an acid value of 0-130mg KOH/g (including gutter oil), and refined vegetable oils such as jatropha oil, rapeseed oil, soybean oil, flax oil, peanut oil, palm oil, and tea seed oil are preferred.

For the transesterification process, according to the process of the present invention, the low carbon alcohol is an aliphatic alcohol having the carbon number of 1-6, and can be a single aliphatic alcohol, or a mixture containing one or more aliphatic alcohols, preferably methanol.

For the transesterification process, according to the process of the present invention, a basic catalyst is used in the transesterification, and said basic catalyst can be one or more of sodium hydroxide, potassium hydroxide, barium hydroxide, calcium hydroxide, magnesium oxide, calcium oxide, barium oxide and diethylamine.

For the transesterification process, according to the process of the present invention, the reaction conditions of the transesterification are as follows: the reaction pressure is 0.5-2.0 MPaG, the reaction temperature is 100-150°C; the molar ratio of low carbon alcohol to triglyceride is 1:3-1:15, and the amount of the basic catalyst is 0.5%-10% by weight of the amount of the oil/fat raw material.

For the transesterification process, according to the process of the present invention, a liquid catalyst is optionally contained in the mass transfer-enhancing material II.

For the transesterification process, according to the process of the present invention, the total residence time in the transesterification reactor is 0.5-7 hours, preferably 0.5-3.5 hours; during this period, the conversion rate is ≥98.5%.

For the transesterification process, according to the process of the present invention, in said microchannel mixing device I, the molar ratio of low carbon alcohol to triglyceride is generally 3:1-15:1, and an appropriately higher alcohol/oil molar ratio can guarantee the mass transfer reaction rate in the transesterification process, but the reactor volume becomes larger, Here, as long as the alcohol/oil molar ratio of ≥3 in the reactor is maintained, it is sufficient. In the microchannel mixing device II, the alcohol/oil molar ratio is generally 1: 10-1:0.33. The alcohol/oil molar ratio in this material is relatively low, that is, the proportion/content of triglyceride is relatively high. As the mass transfer-enhancing material, it can quickly break through the phase interface and transfer to the catalyst surface to supplement the triglyceride consumed in the reaction, keeping a large amount of the homogeneous phase of low carbon alcohol/ triglyceride that is rich in triglyceride molecules on the catalyst surface all along, thereby increasing the transesterification reaction rate and the conversion rate.

For the transesterification process, according to the process of the present invention, in the mixed material I formed by said microchannel mixing device I, the triglyceride droplets have a particle size d1 of 100-900 µm and preferably have the disperse uniformity of ≥80%. At this time, when entering the reactor, no phase separation between the low carbon alcohol phase and the triglyceride phase can be maintained during the reaction residence time, improving the mass transfer reaction rate, and achieving the good reaction effect and the conversion of triglyceride; In the mixed material II formed by microchannel mixing device II, the triglyceride droplets have a particle size d2 of less than 100 µm, preferably 0.1-50 µm.When introduced as a mass transfer-enhancing material into the reactor, due to the small particle size of triglyceride and the concentration of triglyceride molecules, triglyceride molecules can quickly break through the phase interface and transfer to the catalyst surface upon the transesterification, playing a role of further enhancing the reaction mass transfer.

For the transesterification process, according to the process of the present invention, the addition amount of the mixed material II is 1wt%-30wt% of the total materials in the reactor (the total amount of triglyceride and methanol and liquid catalyst); when the mixed material II is divided into multiple streams for the addition, it is preferable to gradually increase the addition amount of each stream along the direction from the inlet to the outlet in the reactor (for example, the addition amount of the latter stream increases by 5-20wt% relative to the addition amount of the former stream). Of course, the low carbon alcohol/ triglyceride molar ratio can also be reduced or unchanged along the direction from the inlet to the outlet in the reactor. Here, as the transesterification proceeds, the triglyceride molecules are gradually consumed, and the mass transfer driving force of the reaction gradually decreases. However, since the amount by mole of low carbon alcohol in the reactor is much greater than that of the triglyceride phase, the low concentration of triglyceride molecules leads to a gradual reduction of triglyceride molecules on the surface of the catalyst. Therefore, by supplementing the mass transfer-enhancing material having a high triglyceride content between the catalyst beds, it can quickly break through the phase interface and transfer to the catalyst surface to timely supplement the triglyceride consumed in the reaction, thereby maintaining a relatively high transesterification reaction rate.

For the transesterification process, according to the process of the present invention, in view of the problems of the oil raw material and methanol being mutually insoluble in the transesterification process of the biodiesel production, easy phase separation in the reaction process, low reaction rate, low conversion rate and long residence time, while the existing technology lacks effective two-phase mixing process and equipment, therefore the present invention provides an efficient reaction apparatus and process for producing biodiesel with transesterification, in which most of the oil/fat raw material and methanol and liquid catalyst are micro-mixed in a certain proportion with the microchannel mixing device I, so that the reaction feed can maintain two phases to be homogeneous without the phase separation during the reaction process in the reactor. In this case, the mole numbers of methanol and liquid catalyst in the reactor are both kept greater than the mole number of triglyceride, thereby achieving the continuous mass transfer between the triglyceride and methanol/ liquid catalyst in the reactor; then a part of triglyceride and methanol and liquid catalyst are micro-mixed in another proportion with the microchannel mixing device II, so that the droplet size (d1) of the triglyceride in the formed mixed material I is less than the droplet size (d2) of the triglyceride phase in the mixed material II, and the droplet concentration degree of triglyceride phase in the mixed material I is less than the droplet concentration degree of the triglyceride phase in the mixed material II, Therefore, when introducing the mixed material II containing a higher concentration degree of the triglyceride phase into the reactor, it can quickly break through the phase interface and promptly supplement the triglyceride consumed in the reaction, playing a role of enhancing the mass transfer, improving the reaction rate of the transesterification and the conversion of raw materials, which is beneficial for reducing the molar ratio of low carbon alcohol/triglyceride, reducing the amount of the reactors or the reaction residence time, and improving the production efficiency of the apparatus for producing biodiesel with transesterification. Here, the droplet concentration degree of the triglyceride refers to the amount of triglyceride micro-droplets dispersed in the mixture of methanol and liquid catalyst per unit volume. At the same time, the special structure of the microchannel components in the microchannel mixing device II, i.e. a certain proportion of metal fiber filaments filled in the crevices between said adjacent sheets, can adhere and spread the water in the mixed feed of triglyceride and methanol and liquid catalyst along the surface of the fiber filaments. The repeated forced cutting by fiber filaments produces micron-sized particles. The fully mixed material contains the concentrated triglyceride droplets. Triglyceride droplets having a smaller size used as the mass transfer-enhancing material can quickly break through the phase interface and promptly supplement oil and fat consumed in the reaction, play a role of enhancing the mass transfer, and reducing the total molar ratio of methanol to triglyceride.

### Brief description of the drawings

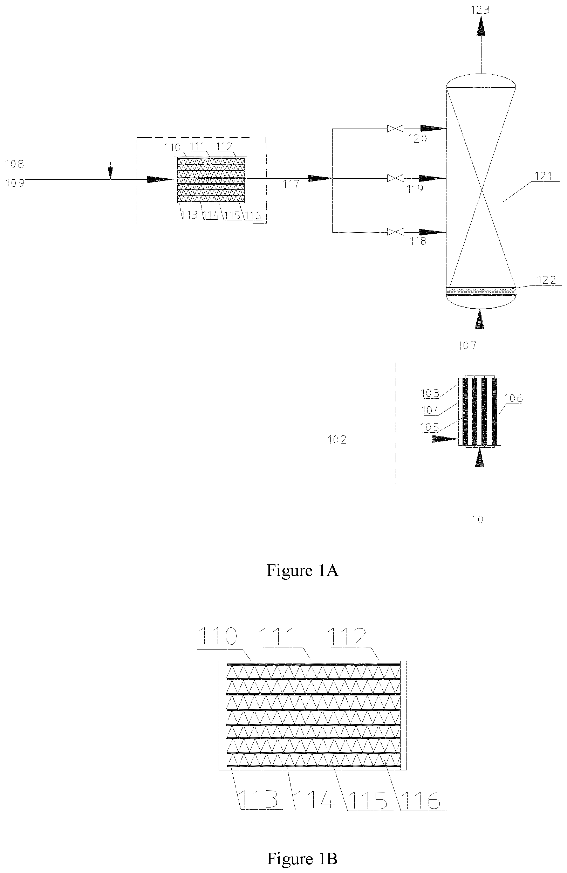

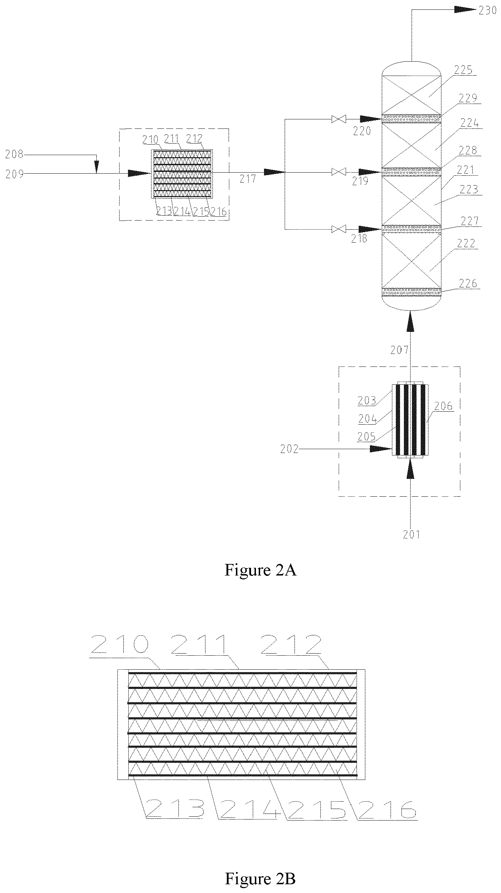

Figure 1A is a schematic diagram of the olefin hydration reaction apparatus of the present invention.
Figure 1B is a schematic diagram of the microchannel component in the microchannel mixing device II,
   In figures 1A and 1B:
   101 Olefin phase I
   102 Aqueous phase I
   103 Microchannel mixing device I
   104 Shell of the tube-shell type inorganic membrane mixer
   105 Bundle of inorganic membrane tubes
   106 Shell space outside the bundle of inorganic membrane tubes
   107 Mixed material I
   108 Olefin phase II
   109 Aqueous phase II
   110 Microchannel mixing device II
   111 Microchannel component
   112 Microchannel shell
   113 Microchannel sheet
   114 Crevice between microchannel sheets
   115 Hydrophilic fiber filaments
   116 Oleophilic fiber filaments
   117 Mixed material II
   118 Mass transfer-enhancing material introduced between the first and second catalyst beds
   119 Mass transfer-enhancing material introduced between the second and third catalyst beds
   120 Mass transfer-enhancing material introduced between the third and fourth catalyst beds
   121 Olefin hydration reactor
   122 First catalyst bed
   123 Second catalyst bed
   124 Third catalyst bed
   125 Fourth catalyst bed
   126 Feed distributor for the first catalyst bed
   127 Feed distributor for the second catalyst bed
   128 Feed distributor for the third catalyst bed
   129 Feed distributor for the fourth catalyst bed
   130 Olefin hydration reaction product
Figure 2A is a schematic diagram of the reaction apparatus for producing biodiesel with transesterification of the present invention.
Figure 2B is a schematic diagram of the microchannel component in the microchannel mixing device II,
   In figures 2A and 2B:
   201 Triglyceride I
   202 Low carbon alcohol and liquid catalyst I
   203 Microchannel mixing device I
   204 Shell of the tube-shell type inorganic membrane mixer
   205 Bundle of inorganic membrane tubes
   206 Shell space outside the bundle of inorganic membrane tubes
   207 Mixed material I
   208 Triglyceride II
   209 Low carbon alcohol and liquid catalyst II
   210 Microchannel mixing device II
   211 Microchannel component
   212 Microchannel shell
   213 Microchannel sheet
   214 Crevice between microchannel sheets
   215 Hydrophilic fiber filaments
   216 Oleophilic fiber filaments
   217 Mixed material II
   218 Mass transfer-enhancing material introduced into the transesterification reactor
   219 Mass transfer-enhancing material introduced into the transesterification reactor
   220 Mass transfer-enhancing material introduced into the transesterification reactor
   221 Transesterification reactor
   222 Material distributor
   223 Transesterification reaction product

### Detailed description

The following provides a detailed explanation of the present invention in conjunction with the accompanying drawings and examples, without limiting the present invention.

Figure 1A is taken as an example to illustrate the olefin hydration reaction apparatus and the olefin hydration process of the present invention:
Firstly, the olefin phase 101 and the aqueous phase 102 are introduced into the microchannel mixing device I 103 in the aqueous phase/olefin phase ratio of ≥1, forming a mixed material I 107. The olefin phase 101 is introduced into the shell space 106 of the microchannel mixing device I 103, and the aqueous phase 102 is introduced into the inorganic membrane tube bundle 105 of the microchannel mixing device I 103. The olefin phase 101 penetrates into the tube through the tube wall of the inorganic membrane tube from the outside. Under the high-speed shear action of water, the two undergo the forced mixing to form the mixed material I 107, As the main reaction material, it enters the olefin hydration reactor 121 and undergoes a reaction in the catalyst bed layer. Another part of olefin phase II 108 and the aqueous phase II 109 are introduced into the microchannel mixing device II 110 at the aqueous phase/olefin phase ratio of <1. After passing through the crevice 114 between the microchannel sheets 113 in the microchannel component 111 provided in the microchannel mixing device II 110, the hydrophilic fiber filaments 115 and the oleophilic fiber filaments 116 filled between the crevice 114 are continuously cut multiple times to form a mixed material II 117, which is introduced as the mass transfer-enhancing material 118 between the first and second catalyst beds, the mass transfer-enhancing material 119 between the second and third catalyst beds, the mass transfer-enhancing material 120 between the third and fourth catalyst beds respectively, between the catalyst beds of the olefin hydration reactor 121, enabling the mass transfer-enhancing material to quickly supplement the olefin molecules consumed during the reaction process, thus achieving the purpose of enhancing the mass transfer. After the completion of the olefin hydration reaction, the reaction product 130 leaves.

The olefin hydration reactor of the present invention is applied to the hydration reactions of propylene, n-butene, isobutene, and cyclohexene, respectively. The specific reaction conditions can be found in Comparative Example 1-1, Comparative Example 1-2, Comparative Example 1-3, Comparative Examples 1-4, Example 1-1, Example 1-2, Example 1-3, Example 1-4, Example 1-5, Example 1-6. The olefin raw materials are commercially available, and their specific properties are shown in Table 1-1, Table 1-2, and Table 1-3. Among them, the catalyst used for propylene hydration is the DIAP type catalyst produced by Dandong Mingzhu Special Resin Co., Ltd., the catalyst used for n-butene hydration is the DNW-II type catalyst produced by Dandong Mingzhu Special Resin Co., Ltd., the catalyst used for isobutene hydration is the DT-017 type catalyst produced by Dandong Mingzhu Special Resin Co., Ltd., and the catalyst used for cyclohexene hydration is the Amberlyst 36 type resin catalyst.

**Table 1-1 Composition of propylene raw material**

| Item | Component | Content, wt% |
|---|---|---|
| 1 | propylene | 95.32 |
| 2 | propane | 3.43 |
| 3 | butane | 0.57 |
| 4 | iso-butane | 0.26 |
| 5 | 2-butene | 0.38 |

**Table 1-2 Composition of n-butene raw material**

| Item | Component | Content, wt% |
|---|---|---|
| 1 | propane | 0.02 |
| 2 | iso-butane | 9.69 |
| 3 | trans-2-butene | 14.22 |
| 4 | cis-2-butene | 5.77 |
| 5 | propylene | 0.01 |
| 6 | n-butane | 11.68 |
| 7 | 1-butene | 58.48 |
| 8 | iso-butene | 0.13 |

**Table 1-3 Composition of isobutene raw material**

| Item | Component | Content, wt% |
|---|---|---|
| 1 | propane | 0.22 |
| 2 | propylene | 0.35 |
| 3 | iso-butane | 0.43 |
| 4 | iso-butene | 98.35 |
| 5 | trans-butene | 0.14 |
| 6 | iso-pentane | 0.15 |
| 7 | cis-butene | 0.25 |
| 8 | Others | 0.11 |

### Comparative Example 1-1

Using a propylene raw material from Table 1-1, isopropanol was prepared by the hydration reaction of propylene with water under the action of a catalyst. The propylene raw material and water were mixed through a conventional static mixer, model SL-1.6/25-10.0-200, and the mixed material entered the propylene hydration reactor for the hydration reaction. The mixing conditions were as follows: the temperature was 155°C, and the pressure was 8.0MPa. The reactor was a regular up-flow reactor, which was equipped with three stages of the catalyst beds. The inlet of each stage of the catalyst bed was equipped with a distribution sieve plate, with a sieve plate hole diameter of 3mm. A mixture of olefin and water was introduced into the bottom of the olefin hydration reactor, evenly distributed along the cross-section of the reactor with the distribution sieve plate, entered the catalyst bed for the olefin hydration reaction, and finally left the olefin hydration reactor through the discharge outlet at the top of the reactor.

The reaction products were obtained from propylene raw material in Table 1-1 through the propylene hydration reactor. The reaction conditions, the residence time, and the raw material conversion rate were shown in Table 1-4.

### Comparative Example 1-2

Using a n-butene raw material from Table 1-2, sec-butanol was prepared by the hydration reaction of n-butene with water under the action of a catalyst. The n-butene raw material and water were mixed three times continuously through a conventional static mixer, model SL-1.6/25-10.0-250, and the mixed material entered the n-butene hydration reactor for the hydration reaction. The mixing conditions were as follows: the temperature was 175°C, and the pressure was 8.0MPa. The reactor was a regular up-flow reactor, which was equipped with four stages of the catalyst beds. The inlet of each stage of the catalyst bed was equipped with a distribution sieve plate, with a sieve plate hole diameter of 2mm. A mixture of n-butene and water was introduced into the bottom of the olefin hydration reactor, evenly distributed along the cross-section of the reactor with the distribution sieve plate, entered the catalyst bed for the olefin hydration reaction, and finally left the olefin hydration reactor through the discharge outlet at the top of the reactor.

The reaction products were obtained from n-butene raw material in Table 1-2 through the n-butene hydration reactor. The reaction conditions, the residence time, and the raw material conversion rate were shown in Table 1-4.

### Comparative Example 1-3

Using an isobutylene raw material from Table 1-3, tert-butanol was prepared by the hydration reaction of isobutene with water under the action of a catalyst. The isobutene raw material and water were mixed through a conventional static mixer, model SL-1.6/25-5.0-200, and the mixed material entered the isobutene hydration reactor for the hydration reaction. The mixing conditions were as follows: the temperature was 105°C, and the pressure was 2.6MPa. The reactor was a regular up-flow reactor, which was equipped with two stages of the catalyst beds. The inlet of each stage of the catalyst bed was equipped with a distribution sieve plate, with a sieve plate hole diameter of 3mm. A mixture of iso-butene and water was introduced into the bottom of the olefin hydration reactor, evenly distributed along the cross-section of the reactor with the distribution sieve plate, entered the catalyst bed for the olefin hydration reaction, and finally left the olefin hydration reactor through the discharge outlet at the top of the reactor.

The reaction products were obtained from iso-butene raw material in Table 1-3 through the iso-butene hydration reactor. The reaction conditions, the residence time, and the raw material conversion rate were shown in Table 1-4.

### Example 1-1

Using a propylene raw material from Table 1-1, isopropanol was prepared by the hydration reaction of propylene with water under the action of a catalyst. A mixed material I was formed by introducing into the microchannel mixing device I at the aqueous phase/olefin phase ratio by mass of 12: 1, and sent as the main reaction material to the olefin hydration reactor for the reaction in the catalyst bed(s). A mixed material II was formed by introducing into the microchannel mixing device II at the aqueous phase/olefin phase ratio by mass of 1:7.5, and introduced as the mass transfer-enhancing material between the catalyst beds to enhance the olefin hydration reaction process. The reaction effluent left the reactor and entered the next separation unit.

The used reactor was the reactor of the present invention, with bottom-in and top-out. Three catalyst beds were provided in the reactor. Two streams of mass transfer-enhancing mixed material II were introduced between the first/second catalyst beds and between the second/third catalyst beds, respectively. The addition amount of mixed material II was 3.6wt% of the total of the materials to the reactor (the total of olefin phase and aqueous phase). The proportion of the mixed material II introduced between the first/second catalyst beds to that introduced between the second/third catalyst beds was 1:1.5. In the microchannel mixing device II, the sheets in the microchannel mixing component were made of stainless steel material and had a thickness of 1.2mm. Five layers of metal fiber filaments having a diameter of 5 µm and one layer of ceramic fiber filaments having a diameter of 5 µm were filled in the crevices between the sheets, wherein the fiber filaments were evenly spaced with a spacing of 1 µm. The fiber filament had a curve shape with periodic changes in wavy lines. The operation conditions of microchannel mixing device I were as follows: the temperature was 150°C, the pressure was 7.5 MPaG; the operation conditions of microchannel mixing device II were as follows: the temperature was 125°C, the pressure was 7.0 MPaG.

The reaction products were obtained from propylene raw material in Table 1-1. The propylene hydration reaction conditions, the residence time, and the raw material conversion rate were shown in Table 1-4.

### Example 1-2

In this Example, the reaction raw materials, the reactor structure, the reaction process, the operation conditions of microchannel mixing device I, and the operation conditions of microchannel mixing device II were identical to those of Example 1-1. Different from Example 1-1, more mild reaction conditions were used in this example. The reaction conditions, the residence time, and the raw material conversion rate were shown in Table 1-4.

### Example 1-3

Using a n-butene raw material from Table 1-2, sec-butanol was prepared by the hydration reaction of n-butene with water under the action of a catalyst. A mixed material I was formed by introducing into the microchannel mixing device I at the aqueous phase/olefin phase ratio by mass of 3:1, and sent as the main reaction material to the olefin hydration reactor for the reaction in the catalyst bed(s). A mixed material II was formed by introducing into the microchannel mixing device II at the aqueous phase/olefin phase ratio by mass of 1:2, and introduced as the mass transfer-enhancing material between the catalyst beds to enhance the olefin hydration reaction process. The reaction effluent left the reactor and entered the next separation unit.

The used reactor was the reactor of the present invention, with bottom-in and top-out. Four catalyst beds were provided in the reactor. The mass transfer-enhancing mixed material II was introduced between the first/second catalyst beds, between the second/third catalyst beds, and between the third/fourth catalyst beds, respectively. The addition amount of mixed material II was 4.0wt% of the total of the materials to the reactor (the total of olefin phase and aqueous phase). The proportion of the mixed material II introduced between the first/second catalyst beds to that introduced between the second/third catalyst beds to that introduced between the third/fourth catalyst beds was 1:1.2:1.5.

In the microchannel mixing device II, the sheets in the microchannel mixing component were made of stainless steel material and had a thickness of 1.0mm. Three layers of glass fiber filaments having a diameter of 1 µm and one layer of ceramic fiber filaments having a diameter of 5 µm were filled in the crevices between the sheets, wherein the fiber filaments were evenly spaced with a spacing of 1 µm. The fiber filament had a curve shape with periodic changes in wavy lines. The operation conditions of microchannel mixing device I were as follows: the temperature was 175°C, the pressure was 7.8 MPaG; the operation conditions of microchannel mixing device II were as follows: the temperature was 135°C, the pressure was 7.0 MPaG.

n-Butene raw material were shown in Table 1-2. The hydration reaction conditions, the residence time, and the raw material conversion rate were shown in Table 1-4.

### Example 1-4

In this Example, the reaction raw materials, the reactor structure, the reaction process, the operation conditions of microchannel mixing device I, and the operation conditions of microchannel mixing device II were identical to those of Example 1-3. Different from Example 1-3, more mild reaction conditions were used in this example. The reaction conditions, the residence time, and the raw material conversion rate were shown in Table 1-4.

### Example 1-5

Using an isobutylene raw material from Table 1-3, tert-butanol was prepared by the hydration reaction of isobutene with water under the action of a catalyst. A mixed material I was formed by introducing into the microchannel mixing device I at the aqueous phase/olefin phase ratio by mass of 4:1, and sent as the main reaction material to the olefin hydration reactor for the reaction in the catalyst bed(s). A mixed material II was formed by introducing into the microchannel mixing device II at the aqueous phase/olefin phase ratio by mass of 1:1.62, and introduced as the mass transfer-enhancing material between the catalyst beds to enhance the olefin hydration reaction process. The reaction effluent left the reactor and entered the next separation unit.

The used reactor was the reactor of the present invention, with bottom-in and top-out. Two catalyst beds were provided in the reactor. The mass transfer-enhancing mixed material II was introduced between the first/second catalyst beds. The addition amount of mixed material II was 2.0wt% of the total of the materials to the reactor (the total of olefin phase and aqueous phase).

In the microchannel mixing device II, the sheets in the microchannel mixing component were made of stainless steel material and had a thickness of 1.5mm. Eight layers of stainless steel fiber filaments having a diameter of 5 µm and two layers of ceramic fiber filaments having a diameter of 5 µm were filled in the crevices between the sheets, wherein the fiber filaments were evenly spaced with a spacing of 1 µm. The fiber filament had a curve shape with periodic changes in wavy lines. The operation conditions of microchannel mixing device I were as follows: the temperature was 105°C, the pressure was 2.8 MPaG; the operation conditions of microchannel mixing device II were as follows: the temperature was 85°C, the pressure was 2.1 MPaG.

Isobutene raw material were shown in Table 1-3. The hydration reaction conditions, the residence time, and the raw material conversion rate were shown in Table 1-4.

### Example 1-6

In this Example, the reaction raw materials, the reactor structure, the reaction process, the operation conditions of microchannel mixing device I, and the operation conditions of microchannel mixing device II were identical to those of Example 1-5. Different from Example 1-5, more mild reaction conditions were used in this example. The reaction conditions, the residence time, and the raw material conversion rate were shown in Table 1-4.

**Table 1-4 Reaction conditions and results**

| No | | Temperature, °C | Pressure, MPaG | VHSP , h⁻¹ | Total mass ratio of aqueous/olefin | Once-through conversion of raw materials, wt% | Selectivity, % |
|---|---|---|---|---|---|---|---|
| 1 | Comparative Examplel-1 | 155-160 | 8.0 | 0.25 | 15 | 64.5 | 92.3 |
| 2 | Comparative Example 1-2 | 175-180 | 8.0 | 1.0 | 2.5 | 6.3 | 96.2 |
| 3 | Comparative Example 1-3 | 105-110 | 2.5 | 1.0 | 3 | 85.6 | 97.5 |
| 4 | Example 1-1 | 150-156 | 7.0 | 0.32 | 10 | 94.7 | 99.3 |
| 5 | Example 1-2 | 130-135 | 6.0 | 0.35 | 10 | 94.2 | 99.3 |
| 6 | Example 1-3 | 175-180 | 7.5 | 1.5 | 2.0 | 50.2 | 98.3 |
| 7 | Example 1-4 | 136-142 | 6.3 | 1.6 | 2.0 | 49.6 | 98.4 |
| 8 | Example 1-5 | 105-111 | 2.5 | 1.2 | 2.2 | 98.2 | 99.2 |
| 9 | Example 1-6 | 100-106 | 2.2 | 1.4 | 2.0 | 97.8 | 99.2 |

The dispersion size and dispersion effect of olefin droplets in water in the process of the present invention were measured by using a high-speed camera, and by selecting several characteristic particles to obtain the particle uniformity of the dispersed phase. The smaller the particle size, the higher the uniformity, the better the dispersion and mixing effect. Therefore, the measurement method for the mixing and dispersion effect in the above Examples and Comparative Examples was to mix the dispersed phase (olefin phase) and the continuous phase (aqueous phase) by using different mixing and dispersion methods (such as conventional static mixer, Microchannel mixing system I and Microchannel mixing system II in the reactor of the present invention) under the same conditions. For each method, at least 10 sets of mixed material samples were obtained, and the UK IX i-SPEED 5 high-speed camera was used to capture the size of the dispersed phase particles in the mixed material samples. The particles in the photo were summed, the percentage contents of the particles of various sizes were calculated to obtain the normal distribution diagram of particles of various sizes, and then obtain the particle uniformity.

From the above examples and comparative examples of the present invention, it could be seen that in case that the olefin hydration reaction apparatus and the reaction process of the present invention are used, due to the introduction of the main reaction material from the bottom of the reactor, the reaction feed maintains two phases to be homogeneous in the reactor, providing a prerequisite for the high conversion rate of the olefin hydration reaction. Then another part of the olefin and water passed through the microchannel mixing device II under the aqueous phase/olefin phase ratio of <1 to form the mixed material II, which was introduced as the mass transfer-enhancing material between catalyst beds. Due to the small size of olefin droplets and the concentrated olefin molecules, the mixed material II can quickly break through the phase interface and supplement the olefin phase consumed in the reaction, greatly enhancing the mass transfer of the entire reaction process, improving the olefin hydration reaction rate and raw material once-through conversion, reducing the aqueous phase/olefin phase ratio, reducing the number of reactors or the reaction residence time, and improving the production efficiency of the olefin hydration apparatus.

Figure 2A is taken as an example to illustrate the reaction apparatus and process for producing biodiesel with transesterification of the present invention:
Firstly, the triglyceride 201 and low carbon alcohol and liquid catalyst 202 are introduced into the microchannel mixing device I 203 in the low carbon alcohol/triglyceride molar ratio of ≥3, forming a mixed material I 207. The triglyceride 201 is introduced into the shell space 206 of the microchannel mixing device I 203, and the low carbon alcohol and liquid catalyst 202 is introduced into the inorganic membrane tube bundle 205 of the microchannel mixing device I 203. The triglyceride 201 penetrates into the tube through the tube wall of the inorganic membrane tube from the outside. Under the high-speed shear action of the low carbon alcohol and liquid catalyst, the two undergo the forced mixing to form the mixed material I 207, As the main reaction material, it enters the transesterification reactor 221 and undergoes the transesterification reaction in the reactor. Another part of triglyceride II 208 and the low carbon alcohol and liquid catalyst II 209 are introduced into the microchannel mixing device II 210 at the low carbon alcohol/ triglyceride molar ratio of <3. After passing through the crevice 214 between the microchannel sheets 213 in the microchannel component 211 provided in the microchannel mixing device II 210, the hydrophilic fiber filaments 215 and the oleophilic fiber filaments 216 filled between the crevice 214 are continuously cut multiple times to form a mixed material II 217, which is introduced as the mass transfer-enhancing material 218, the mass transfer-enhancing material 219, and the mass transfer-enhancing material 220 respectively into the transesterification reactor 221, enabling the mass transfer-enhancing materials to quickly supplement the triglyceride consumed during the reaction process, thus achieving the purpose of enhancing the mass transfer. After the completion of the transesterification reaction, the reaction product 222 leaves.

The raw material triglyceride used in the examples and comparative examples of the present invention is tung oil, and its properties are shown in Table 1.

**Table 1: Properties of raw material**

| No | Item | Index |
|---|---|---|
| 1 | Color (Lovibond colorimeter 1 inch cuvette) | Yellow: 35, Red: ≤3.0 |
| 2 | Odor | Inherent normal odor of tung oil, without off-flavor |
| 3 | Transparency (stood for 24 hours, 20°C) | Transparent |
| 4 | Acid value/mg(KOH)·g⁻¹ | 3.0 |
| 5 | Moisture and volatile matter, % | 0.10 |
| 6 | Mechanical impurity,% | No |

### Comparative Example 2-1

The conventional transesterification apparatus and reaction process for biodiesel products were used. The raw materials for transesterification were the oil/fat raw material, methanol and basic catalyst (see Table 1 for the properties of the oil/fat raw material). First, the oil/fat raw material, methanol and basic catalyst were introduced into a stirred tank for stirring and mixing for 15-20 minutes, and then pumped with a feeding pump into a two-stage reactor to perform the transesterification, wherein the first stage reactor was a column reactor with a size of ϕ200×1200mm, the second stage reactor was a tube reactor with a size of ϕ80×12800mm.

The operation conditions of the reactor were as follows:
Feeding rate of the oil/fat raw material: 1.5kg/h;
The reaction temperature was 120°C-125°C;
The reaction pressure was 2.0 MPaG;
Alcohol/oil molar ratio: 8-10 (the molecular weight of the oil and fat was equivalent to 880, the same for the following)
The mass fraction of basic catalyst relative to the oil/fat raw material: 2.5%.

Under the reaction conditions, the raw material conversion at the outlet of the first stage reactor was 75.2%, the raw material conversion at the outlet of the second stage reactor was 87.4%; the first-stage transesterification residence time (based on the total of the materials) was 2.02 hours, the second-stage transesterification residence time (based on the total of the materials) was 3.44 hours.

### Comparative Example 2-2

The conventional transesterification apparatus and reaction process for biodiesel products were used. The raw materials for transesterification were the oil/fat raw material, methanol and basic catalyst (see Table 1 for the properties of the oil/fat raw material). First, the oil/fat raw material, methanol and basic catalyst were introduced into a collision-type reactor for mixing by collision for 5-10 minutes, and then pumped with a feeding pump into a two-stage reactor to perform the transesterification, wherein the first stage reactor was a column reactor with a size of ϕ200×1200mm, the second stage reactor was a tube reactor with a size of ϕ80×12800mm.

The operation conditions of the reactor were as follows:
Feeding rate of the oil/fat raw material: 1.8kg/h;
The reaction temperature was 120°C-125°C;
The reaction pressure was 2.0 MPaG;
Alcohol/oil molar ratio: 8-10 (the molecular weight of the oil and fat was equivalent to 880)
The mass fraction of basic catalyst relative to the oil/fat raw material: 2.5%.

Under the reaction conditions, the raw material conversion at the outlet of the first stage reactor was 87.2%, the raw material conversion at the outlet of the second stage reactor was 90.5%; the first-stage transesterification residence time (based on the total of the materials) was 1.68 hours, the second-stage transesterification residence time (based on the total of the materials) was 2.87 hours.

### Example 2-1

The transesterification process of the present invention was used. The raw materials for transesterification were the oil/fat raw material, methanol and basic catalyst (see Table 1 for the properties of the oil/fat raw material). First, the oil/fat raw material, methanol and basic catalyst were introduced to the microchannel mixing device I, wherein the molar ratio of the oil/fat raw material/methanol was 6: 1, the oil/fat raw material was introduced into the shell side of the microchannel mixing device I, and methanol and liquid basic catalyst were introduced into the tube side of the microchannel mixing device I, the mixed material I formed with the microchannel mixing device I was used as the main reaction material to enter a two-stage transesterification reactor to perform the transesterification, wherein the first stage reactor was a column reactor with a size of ϕ200×800mm, the second stage reactor was a tube reactor with a size of ϕ80×6400mm a mixed material II was formed from the oil/fat raw material and methanol in the molar ratio of 1:1 with the microchannel mixing device II, and introduced as the mass transfer-enhancing material into the column reactor and the tubular reactor respectively to enhance the transesterification process. The reaction effluent left the reactor and entered the next separation unit.

In the microchannel mixing device II, the sheets in the microchannel mixing component were made of stainless steel material and had a thickness of 1.2mm. Five layers of metal fiber filaments having a diameter of 5 µm and one layer of ceramic fiber filaments having a diameter of 5 µm were filled in the crevices between the sheets, wherein the fiber filaments were evenly spaced with a spacing of 1 µm.The fiber filament had a curve shape with periodic changes in wavy lines.

The operation conditions of the transesterification process were as follows:
Feeding rate of the oil/fat raw material: 3.6kg/h;
Reaction temperature: 120°C-125°C;
Reaction pressure: 2.0 MPaG;
The mass fraction of basic catalyst relative to the oil/fat raw material: 2.5%;
The mass transfer-enhancing material added to the column reactor comprised 25.6wt% of the total reaction feed, while the mass transfer-enhancing material added to the tube reactor comprised 5.2wt% of the total reaction feed.

The operation conditions of microchannel mixing device I were as follows: the temperature was 120-125°C, the pressure was 2.0 MPaG; the operation conditions of microchannel mixing device II were as follows: the temperature was 120°C, the pressure was 2.0 MPaG.

Under the reaction conditions, the raw material conversion in the first-stage transesterification was 96.30%, the raw material conversion in the second-stage transesterification was 98.7%; the first-stage transesterification residence time was 0.87 hours, the second-stage transesterification residence time was 1.11 hours.

### Example 2-2

In this Example, the reaction raw materials, the reactor structure, the reaction process, the operation conditions of microchannel mixing device I, and the operation conditions of microchannel mixing device II were identical to those of Example 2-1. Different from Example 2-1, in this example, on the one hand, the transesterification conditions were changed, on the other hand, the introduction position and amount of the mass transfer-enhancing material were appropriately adjusted.

The operation conditions of the transesterification process were as follows:
Feeding rate of the oil/fat raw material: 3.6kg/h;
Reaction temperature: 120°C-125°C;
Reaction pressure: 1.8 MPaG;
The mass fraction of basic catalyst relative to the oil/fat raw material: 2.5%;
The mass transfer-enhancing material added to the column reactor comprised 16.6wt% of the total reaction feed, while the mass transfer-enhancing material added to the tube reactor comprised 8.0wt% of the total reaction feed.

Under the reaction conditions, the raw material conversion in the first-stage transesterification was 97.10%, the raw material conversion in the second-stage transesterification was 98.8%; the first-stage transesterification residence time was 0.87 hours, the second-stage transesterification residence time was 1.11 hours.

### Example 2-3

The transesterification process of the present invention was used. The raw materials for transesterification were the oil/fat raw material, methanol and basic catalyst (see Table 1 for the properties of the oil/fat raw material). First, the oil/fat raw material, methanol and basic catalyst were introduced to the microchannel mixing device I, wherein the molar ratio of the oil/fat raw material/methanol was 5: 1, the oil/fat raw material was introduced into the shell side of the microchannel mixing device I, and methanol and liquid basic catalyst were introduced into the tube side of the microchannel mixing device I, the mixed material I formed with the microchannel mixing device I was used as the main reaction material to enter a two-stage transesterification reactor to perform the transesterification, wherein the first stage reactor was a column reactor with a size of ϕ200×800mm, the second stage reactor was a tube reactor with a size of ϕ80×6400mm; a mixed material II was formed from the oil/fat raw material and methanol in the molar ratio of 1:2 with the microchannel mixing device II, and introduced as the mass transfer-enhancing material into the column reactor and the tubular reactor respectively to enhance the transesterification process. The reaction effluent left the reactor and entered the next separation unit.

In the microchannel mixing device II, the sheets in the microchannel mixing component were made of stainless steel material and had a thickness of 1.0mm. Three layers of glass fiber filaments having a diameter of 1 µm and one layer of ceramic fiber filaments having a diameter of 5 µm were filled in the crevices between the sheets, wherein the fiber filaments were evenly spaced with a spacing of 1 µm.The fiber filament had a curve shape with periodic changes in wavy lines.

The operation conditions of the transesterification process were as follows:
Feeding rate of the oil/fat raw material: 3.6kg/h;
Reaction temperature: 120°C-125°C;
Reaction pressure: 2.0 MPaG;
The mass fraction of basic catalyst relative to the oil/fat raw material: 2.5%;
The mass transfer-enhancing material added to the column reactor comprised 20.0wt% of the total reaction feed, while the mass transfer-enhancing material added to the tube reactor comprised 3.6wt% of the total reaction feed.

The operation conditions of microchannel mixing device I were as follows: the temperature was 120-125°C, the pressure was 2.0 MPaG; the operation conditions of microchannel mixing device II were as follows: the temperature was 120°C, the pressure was 2.0 MPaG.

Under the reaction conditions, the raw material conversion in the first-stage transesterification was 97.0%, the raw material conversion in the second-stage transesterification was 98.9%; the first-stage transesterification residence time was 1.01 hours, the second-stage transesterification residence time was 1.30 hours.

### Example 2-4

In this Example, the reaction raw materials, the reactor structure, the reaction process, the operation conditions of microchannel mixing device I, and the operation conditions of microchannel mixing device II were identical to those of Example 2-3. Different from Example 2-3, in this example, on the one hand, the feed rate and the transesterification conditions were changed, on the other hand, the introduction position and amount of the mass transfer-enhancing material were appropriately adjusted.

The operation conditions of the transesterification process were as follows:
Feeding rate of the oil/fat raw material: 4.0kg/h;
Reaction temperature: 120°C-125°C;
Reaction pressure: 2.0 MPaG;
The mass fraction of basic catalyst relative to the oil/fat raw material: 3.0%;
The mass transfer-enhancing material added to the column reactor comprised 22.4wt% of the total reaction feed, while the mass transfer-enhancing material added to the tube reactor comprised 3.2wt% of the total reaction feed.

Under the reaction conditions, the raw material conversion in the first-stage transesterification was 96.8%, the raw material conversion in the second-stage transesterification was 99.1%; the first-stage transesterification residence time was 1.01 hours, the second-stage transesterification residence time was 1.30 hours.

### Example 2-5

The transesterification process of the present invention was used. The raw materials for transesterification were the oil/fat raw material, methanol and basic catalyst (see Table 1 for the properties of the oil/fat raw material). First, the oil/fat raw material, methanol and basic catalyst were introduced to the microchannel mixing device I, wherein the molar ratio of the oil/fat raw material/methanol was 8: 1, the oil/fat raw material was introduced into the shell side of the microchannel mixing device I, and methanol and liquid basic catalyst were introduced into the tube side of the microchannel mixing device I, the mixed material I formed with the microchannel mixing device I was used as the main reaction material to enter a two-stage transesterification reactor to perform the transesterification, wherein the first stage reactor was a column reactor with a size of ϕ200×800mm, the second stage reactor was a tube reactor with a size of ϕ80×6400mm; a mixed material II was formed from the oil/fat raw material and methanol in the molar ratio of 1:1 with the microchannel mixing device II, and introduced as the mass transfer-enhancing material into the column reactor and the tubular reactor respectively to enhance the transesterification process. The reaction effluent left the reactor and entered the next separation unit.

In the microchannel mixing device II, the sheets in the microchannel mixing component were made of stainless steel material and had a thickness of 1.5mm. Eight layers of stainless steel fiber filaments having a diameter of 5 µm and two layers of ceramic fiber filaments having a diameter of 5 µm were filled in the crevices between the sheets, wherein the fiber filaments were evenly spaced with a spacing of 1 µm.The fiber filament had a curve shape with periodic changes in wavy lines.

The operation conditions of the transesterification process were as follows:
Feeding rate of the oil/fat raw material: 4.0kg/h;
Reaction temperature: 120°C-125°C;
Reaction pressure: 2.0 MPaG;
The mass fraction of basic catalyst relative to the oil/fat raw material: 2.5%;
The mass transfer-enhancing material added to the column reactor comprised 21.6wt% of the total reaction feed, while the mass transfer-enhancing material added to the tube reactor comprised 1.5wt% of the total reaction feed.

The operation conditions of microchannel mixing device I were as follows: the temperature was 120-125°C and a pressure of 2.0 MPaG; the operation conditions of microchannel mixing device II were as follows: the temperature was 120°C, and the pressure was 2.0 MPaG.

Under the reaction conditions, the raw material conversion in the first-stage transesterification was 97.8%, the raw material conversion in the second-stage transesterification was 98.9%; the first-stage transesterification residence time was 0.611 hours, the second-stage transesterification residence time was 0.782 hours.

### Examples 2-6

In this Example, the reaction raw materials, the reactor structure, the reaction process, the operation conditions of microchannel mixing device I, and the operation conditions of microchannel mixing device II were identical to those of Example 2-5. Different from Example 2-5, in this example, on the one hand, the feed rate and the transesterification conditions were changed, on the other hand, the introduction position and amount of the mass transfer-enhancing material were appropriately adjusted.

The operation conditions of the transesterification process were as follows:
Feeding rate of the oil/fat raw material: 4.0kg/h;
Reaction temperature: 120°C-125°C;
Reaction pressure: 1.8 MPaG;
The mass fraction of basic catalyst relative to the oil/fat raw material: 3.0%;
The mass transfer-enhancing material added to the column reactor comprised 17.5wt% of the total reaction feed, while the mass transfer-enhancing material added to the tube reactor comprised 6.4wt% of the total reaction feed.

Under the reaction conditions, the raw material conversion in the first-stage transesterification was 97.3%, the raw material conversion in the second-stage transesterification was 98.8%; the first-stage transesterification residence time was 0.611 hours, the second-stage transesterification residence time was 0.782 hours.

The dispersion size and dispersion effect of triglyceride droplets in low carbon alcohol in the process of the present invention were measured by using a high-speed camera, and by selecting several characteristic particles to obtain the particle uniformity of the dispersed phase. The smaller the particle size, the higher the uniformity, the better the dispersion and mixing effect.

Therefore, the measurement method for the mixing and dispersion effect in the above Examples and Comparative Examples was to mix the dispersed phase (triglyceride) and the continuous phase (low carbon alcohol phase) by using different mixing and dispersion methods (such as conventional static mixer, microchannel mixing system I and microchannel mixing system II in the reactor of the present invention) under the same conditions. For each method, at least 10 sets of mixed material samples were obtained, and the UK IX i-SPEED 5 high-speed camera was used to capture the size of the dispersed phase particles in the mixed material samples. The particles in the photo were summed, the percentage contents of the particles of various sizes were calculated to obtain the normal distribution diagram of particles of various sizes, and then obtain the particle uniformity.

From the above examples and comparative examples of the present invention, it could be seen that in case that the apparatus and process for producing biodiesel by transesterification of the present invention were used, due to the introduction of the mixed material I formed from oil/fat raw material, low carbon alcohol and catalyst with the microchannel mixing device I as the main reaction material into one end of the reactor, wherein the molar ratio of the low carbon alcohol to the oil/fat raw material in the mixed material I was ≥3,the reaction feed maintained two phases to be homogeneous in the reactor, providing a prerequisite for the high conversion rate of the transesterification reaction. Then another part of the oil/fat raw material having a molar ratio of low carbon alcohol to triglyceride of ≥3,and methanol and catalyst passed through the microchannel mixing device II under the molar ratio of low carbon alcohol to triglyceride of <1 to form the mixed material II, which was introduced as the mass transfer-enhancing material into the transesterification reactor. Due to the small size of the oil/fat raw material droplets and the concentrated molecules, the mixed material II could quickly break through the phase interface and supplement the oil/fat raw material consumed in the reaction, greatly enhancing the mass transfer of the entire reaction process, improving the transesterification reaction rate and raw material once-through conversion, reducing the molar ratio of low carbon alcohol to triglyceride, reducing the number of reactors or the reaction residence time, and improving the production efficiency of the apparatus for producing biodiesel with transesterification.

## Claims

1. A microchannel liquid-liquid mixing device, comprising a microchannel component and a shell, wherein the microchannel component is fixed inside the shell, wherein an inlet is provided at one end of the shell for feeding at least two reaction liquid phases, and an outlet is provided at the other end for discharging a mixed material; said microchannel component comprises multiple stacked sheets and oleophilic and hydrophilic fiber filaments filled in the crevices between adjacent sheets, wherein the fiber filaments form several microchannels between them, and the fiber filaments are clamped and fixed by the sheets.

2. The microchannel liquid-liquid mixing device according to any of the aforementioned claims, which is **characterized in that**: the microchannel component in the shell of the microchannel mixer are divided into a feeding end and a discharging end along the direction of the crevice, wherein a feeding distribution space is provided between the material inlet and the feeding end, and a discharging distribution space is provided between the material outlet and the discharging end, except for the feeding end and the discharging end, all other ends of the microchannel component are connected to the shell in a sealed manner.

3. The microchannel liquid-liquid mixing device according to any of the aforementioned claims, which is **characterized in that**: said fiber filaments can be arranged in single or multiple layers, preferably 1-50 layers, and more preferably 1-5 layers.

4. The microchannel liquid-liquid mixing device according to any of the aforementioned claims, which is **characterized in that**: when said fiber filaments are arranged in multiple layers, the projection of two adj acent layers of fiber filaments along the vertical direction of the sheets forms a mesh structure.

5. The microchannel liquid-liquid mixing device according to any of the aforementioned claims, which is **characterized in that**: in any layer, preferably, in each layer of fiber filaments, the distance between adjacent fiber filaments is 0.5 µm-50 µm, preferably arranged at equal intervals; and/or, the fiber filaments are arranged along any of the transverse, longitudinal or oblique direction of the surface of the sheet.

6. The microchannel liquid-liquid mixing device according to any of the aforementioned claims, which is **characterized in that**: said fiber filament has an arbitrary curve shape, preferably a periodically changing curve shape.

7. The microchannel liquid-liquid mixing device according to any of the aforementioned claims, which is **characterized in that**: the fiber filaments in the same layer have the same shape, and preferably, the fiber filaments in all layers have the same shape.

8. The microchannel liquid-liquid mixing device according to any of the aforementioned claims, which is **characterized in that**: said fiber filaments have a diameter of 0.5-50 µm, preferably 0.5-5 µm, more preferably 0.5-1 µm.

9. The microchannel liquid-liquid mixing device according to any of the aforementioned claims, which is **characterized in that**: said oleophilic fiber filament is at least one of a polyester fiber filament, a nylon fiber filament, a stainless steel fiber filament, a polyurethane fiber filament, a polypropylene fiber filament, a polyacrylonitrile fiber filament, a polyvinyl chloride fiber filament, or an oleophilically surface-treated fiber filament material.

10. The microchannel liquid-liquid mixing device according to any of the aforementioned claims, which is **characterized in that**: said hydrophilic fiber filament is selected from one or more of a high molecular polymer containing at least one hydrophilic group in its main chain or side chain or a fiber filament that has been hydrophilically treated with a physical or chemical method.

11. The microchannel liquid-liquid mixing device according to any of the aforementioned claims, which is **characterized in that**: said hydrophilic fiber filament is selected from one or more of glass fiber filament, ceramic fiber filament, polypropylene fiber, polyamide fiber or acrylic fiber.

12. The microchannel liquid-liquid mixing device according to any of the aforementioned claims, which is **characterized in that**: said sheet has a thickness of 0.05mm-5mm, preferably 0.1-1.5mm.

13. The microchannel liquid-liquid mixing device according to any of the aforementioned claims, which is **characterized in that**: the ratio by weight of the oleophilic fiber filament to the hydrophilic fiber filament filled in the crevices between said adjacent sheets is 1:50-1:1.

14. The microchannel liquid-liquid mixing device according to any of the aforementioned claims, which is **characterized in that**: the hydrophilic fiber filaments in any layer are uniformly distributed in the oleophilic fiber filaments.

15. The microchannel liquid-liquid mixing device according to any of the aforementioned claims, which is **characterized in that**: the ratio by weight of the oleophilic fiber filament to the hydrophilic fiber filament in any layer is 1:50-1:1.

16. The microchannel liquid-liquid mixing device according to any of the aforementioned claims, which is **characterized in that**: the sheet is of any one or more of metal, ceramics, organic glass, or polyester material.

17. The microchannel liquid-liquid mixing device according to any of the aforementioned claims, which is **characterized in that**: the shape of the sheet is any one of rectangle, square, polygon, circle, ellipse, or sector.

18. A liquid-liquid reaction apparatus, which includes a microchannel mixing device I, a microchannel mixing device II, and a reactor;
said microchannel mixing device I has a tube-shell type structure, and a bundle of inorganic membrane tubes is arranged inside the shell; the inlet end of the bundle of inorganic membrane tubes is communicated with the first liquid phase feeding pipeline, the cavity in the shell outside of the bundle of inorganic membrane tubes is communicated with a second liquid phase feeding pipeline, and the outlet end of the bundle of inorganic membrane tubes is an outlet for a mixed material I; the microchannel mixing device I is used for feeding a first liquid phase and a second liquid phase to form a mixed material I, the second liquid phase diffuses into the first liquid phase inside the inorganic membrane tube through porous channels in the tube wall of the inorganic membrane tube from the cavity in the shell, and under the action of the shearing force of the first liquid phase having a high flow rate in the tube, two liquid phases forms a homogeneous mixed material I, which is used as the main reaction feed; preferably, a control device is provided in the microchannel mixing device I so that the ratio of the first liquid phase to the second liquid phase is greater than or less than (preferably greater than) the theoretical ratio of the first liquid phase to the second liquid phase of the reaction;
said microchannel mixing device II is a microchannel liquid-liquid mixing device according to claim 1, which includes a microchannel component and a shell, the microchannel component is fixed inside the shell, an inlet is provided at one end of the shell for feeding the first liquid phase and the second liquid phase, and an outlet is provided at the other end for discharging a mixed material II; said microchannel component comprises multiple stacked sheets and oleophilic and hydrophilic fiber filaments filled in the crevices between adjacent sheets, wherein the fiber filaments form several microchannels between them, and the fiber filaments are clamped and fixed by the sheets; the microchannel mixing device II is used for the first liquid phase and the second liquid phase to form the mixed material II, and the first liquid phase and the second liquid phase are cut by fiber filaments and mixed in the microchannel mixing device II to form the mixed material II; wherein preferably a control device is provided in the microchannel mixing device II so that the ratio of the first liquid phase to the second liquid phase is not greater than or not less than (preferably not greater than) the theoretical ratio of the first liquid phase to the second liquid phase of the reaction;
the top, the bottom, or the side of the reactor is provided with feed inlet(s), while the bottom, the top, or the side is provided with discharge outlet(s); the reactor body is provided with an inlet for the mass transfer-enhancing material; in principle, the inlet for the mass transfer-enhancing material can be provided at any position within the reactor; the outlet for the mixed material I of the microchannel mixing device I is connected to the feed inlet through pipeline(s), and the outlet for the mixed material II of the microchannel mixing device II is connected to the inlet for the mass transfer-enhancing material.

19. The liquid-liquid reaction apparatus according to claim 18, which is **characterized in that**: the bundle of inorganic membrane tubes of said microchannel mixing device I is of one or more of ceramic membrane, metal membrane, metal/ceramic composite membrane, alloy membrane, molecular sieve composite membrane, zeolite membrane glass membrane or the like; the tube wall of the inorganic membrane tube has a hole diameter of 10nm-1 µm.

20. An olefin hydration reaction process by using the liquid-liquid reaction apparatus according to claim 18, which is **characterized in that** said olefin hydration reactor is a fix bed reactor, the inlet for the mass transfer-enhancing material is provided between two adjacent catalyst beds; the catalyst bed is filled with an olefin hydration catalyst; one or more olefin hydration reactors can be provided as required, when more than one reactor is provided, the reactors are connected in parallel or in series; one or more catalyst bed(s) are provided in the reactor.

21. The olefin hydration reaction according to claim 20, which is **characterized in that**: a mixed material I is formed by mixing an olefin phase and an aqueous phase with an aqueous phase/olefin phase ratio of ≥1 with the microchannel mixing device I and sent to the bottom of the olefin hydration reactor as the main reaction material; and a mixed material II is formed by mixing an olefin phase and an aqueous phase with an aqueous phase/olefin phase ratio of <1 with a microchannel mixing device II and introduced to the reactor as the mass transfer-enhancing material; the mixed material I and the mixed material II undergo the olefin hydration reaction in the catalyst bed(s), and the reaction products flow out from the outlet at the top of the reactor and enter the next separation unit.

22. The olefin hydration reaction process according to claim 20, which is **characterized in that** the operation conditions of microchannel mixing device I generally are as follows: the temperature is normal temperature to 250°C, pressure is 1.0-10.0 MPaG; the operation conditions of microchannel mixing device II generally are as follows: the temperature is normal temperature to 200°C, the pressure is 1.0-10.0 MPaG.

23. The olefin hydration reaction process according to claim 20, which is **characterized in that** the olefin phase is generally any one of ethylene, propylene, n-butene, isobutene, isopentene, cyclohexene or the like.

24. The olefin hydration reaction process according to claim 20, which is **characterized in that** the olefin hydration reactor generally adopts a form of bottom-in and top-out.

25. The olefin hydration reaction process according to claim 20, which is **characterized in that** in the microchannel mixing device I, the aqueous phase/olefin phase ratio by mass is generally 2:1-20:1, and in the microchannel mixing device II, the aqueous phase/olefin phase ratio by mass is generally 1:20-1:1.

26. The olefin hydration reaction process according to claim 20, which is **characterized in that** in the mixed material I formed by said microchannel mixing device I, the olefin droplets have a particle size d1 of 100-900 µm and preferably have the disperse uniformity of ≥80%;
in the mixed material II formed by said microchannel mixing device II, the olefin droplets have a particle size d2 of less than 100 µm and preferably 0.1-50 µm.

27. The olefin hydration reaction process according to claim 20, which is **characterized in that** the addition amount of the mixed material II is 1wt%-30wt% of the total materials in the reactor (the total amount of the olefin phase and the aqueous phase); when the mixed material II is divided into multiple streams for the addition, it is preferable to gradually increase the addition amount of each stream along the flow direction of the materials in the reactor (for example, the addition amount of the latter stream increases by 5-20wt% relative to the addition amount of the former stream); and/or, preferably the aqueous phase/olefin phase ratio along the flow direction of the materials in the reactor is reduced or unchanged.

28. The olefin hydration reaction process according to claim 20, which is **characterized in that** a catalyst with acid catalytic function, such as mineral acid, benzene sulfonic acid, ion exchange resin, molecular sieve, and other types of catalysts, is generally used in the catalyst bed(s) of the olefin hydration reactor.

29. The olefin hydration reaction process according to claim 20, which is **characterized in that** the conditions of the olefin hydration reaction is generally as follows: the temperature is 80-250°C, the pressure is 1.0-10.0 MPaG, and the space velocity is 0.1-3.0 h⁻¹.

30. A transesterification process performed by using the liquid-liquid reaction device according to claim 18, which is **characterized in that** the reactor is a tank reactor, a column reactor, a tubular reactor, or an improved form of the aforementioned reactors; one or more reactors can be provided as required, and the reactors can be connected in parallel or series; at least one mixed material formed by the microchannel mixing device II is introduced as the mass transfer-enhancing material.

31. The transesterification process according to claim 30, which is **characterized in that**: a mixed material I is formed by mixing two phases of low carbon alcohol and triglyceride having the molar ratio of low carbon alcohol to triglyceride of ≥3 with the microchannel mixing device I and sent to the transesterification reactor as the main reaction material; and a mixed material II is formed by mixing two phases of low carbon alcohol and triglyceride having the molar ratio of low carbon alcohol to triglyceride of <3 with a microchannel mixing device II and introduced to the reactor as the mass transfer-enhancing material; the mixed material I and the mixed material II undergo the transesterification reaction in the reactor, and the reaction products flow out from the outlet of the reactor and enter the separation unit.

32. The transesterification process according to claim 30, which is **characterized in that**: the operation conditions of the microchannel mixing device I generally include: the temperature is normal temperature to 150°C, the pressure is 0.5-3.0 MPaG; the operation conditions of the microchannel mixing device II generally include: the temperature is normal temperature to 150°C, the pressure is 0.5-3.0 MPaG, in particular, the amount of the liquid catalyst is 0.5%-10% of the amount of the oil/fat raw material.

33. The transesterification process according to claim 30, which is **characterized in that**: the oil/fat raw material is a triglyceride, mainly derived from animal oils or vegetable oils, including the oil and fat having an acid value of 0-130mg KOH/g (including gutter oil), and refined vegetable oils such as jatropha oil, rapeseed oil, soybean oil, flax oil, peanut oil, palm oil, and tea seed oil are preferred.

34. The transesterification process according to claim 30, which is **characterized in that**: the low carbon alcohol is an aliphatic alcohol having the carbon number of 1-6, and can be a single aliphatic alcohol, or a mixture containing one or more aliphatic alcohols, preferably methanol.

35. The transesterification process according to claim 30, which is **characterized in that**: a basic catalyst is used in the transesterification, and said basic catalyst can be one or more of sodium hydroxide, potassium hydroxide, barium hydroxide, calcium hydroxide, magnesium oxide, calcium oxide, barium oxide and diethylamine.

36. The transesterification process according to claim 30, which is **characterized in that**: the reaction conditions of the transesterification are as follows: the reaction pressure is 0.5-2.0 MPaG, the reaction temperature is 100-150°C; the molar ratio of low carbon alcohol to triglyceride is 1:3-1:15, and the amount of the basic catalyst is 0.5%-10% by weight of the amount of the oil/fat raw material.

37. The transesterification process according to claim 30, which is **characterized in that**: a liquid catalyst is optionally contained in the mass transfer-enhancing material II.

38. The transesterification process according to claim 30, which is **characterized in that**: the total residence time in the transesterification reactor is 0.5-7 hours, preferably 0.5-3.5 hours.

39. The transesterification process according to claim 30, which is **characterized in that**: in the microchannel mixing device I, the low carbon alcohol/triglyceride molar ratio is generally 3:1 - 15:1, and in the microchannel mixing device II, the alcohol/oil molar ratio is generally 1:10-1:0.33.

40. The transesterification process according to claim 30, which is **characterized in that**: in the mixed material I formed by said microchannel mixing device I, the triglyceride droplets have a particle size d1 of 100-900 µm and preferably have the disperse uniformity of ≥80%; and in the mixed material II formed by said microchannel mixing device II, the triglyceride droplets have a particle size d2 of less than 100 µm and preferably 0.1-50 µm.
